# FASCICULE DE BREVET EUROPEEN

(11) **EP 4 262 753 B1**
(45) Date de publication et mention de la délivrance du brevet: **05.02.2025**
(21) Numéro de dépôt: 21854935.0
(22) Date de dépôt: 17.12.2021
(51) Int. Cl.: A61K 9/06, A61K 31/167, A61K 47/32, A61K 47/36, A61P 23/02, A61K 9/00, A61K 9/70

(54) **PRODUIT PHARMACEUTIQUE, SON PROCÉDÉ DE PRÉPARATION ET SES UTILISATIONS**
PHARMAZEUTISCHES PRODUKT, VERFAHREN ZUR HERSTELLUNG DAVON UND VERWENDUNGEN DAVON
PHARMACEUTICAL PRODUCT, METHOD FOR PREPARING SAME AND USES THEREOF

(30) Priorité: 18.12.2020 FR 2013642
(43) Date de publication de la demande: 25.10.2023
(73) Titulaire: Commissariat à l'Energie Atomique et aux Energies Alternatives, 75015 Paris (FR)
(72) Inventeur: TEXIER-NOGUES, Isabelle, 38054 GRENOBLE CEDEX 09 (FR); JARY, Dorothée, 38054 GRENOBLE CEDEX 09 (FR); LAURO, Dominique, 38054 GRENOBLE CEDEX 09 (FR); VIGNOUD, Séverine, 38054 GRENOBLE CEDEX 09 (FR)
(74) Mandataire: Brevalex
(86) Numéro de dépôt international: PCT/FR2021/052399
(87) Numéro de publication internationale: WO 2022/129817

(56) Documents cités:
- WO-A1-89/10740
- WO-A1-98/17263
- US-A1- 2003 220 403
- US-A1- 2015 216 796
- KIM JUNG DONG ET AL: "Droplet-born air blowing: Novel dissolving microneedle fabrication", JOURNAL OF CONTROLLED RELEASE, ELSEVIER, AMSTERDAM, NL, vol. 170, no. 3, 3 June 2013 (2013-06-03), pages 430 - 436, XP028691879, ISSN: 0168-3659, DOI: 10.1016/J.JCONREL.2013.05.026

## Description

### DOMAINE TECHNIQUE

La présente invention appartient au domaine général des produits pharmaceutiques et notamment des produits pharmaceutiques permettant une diffusion passive de substances actives au niveau topique, transdermique, transgingivale et/ou transmucosale.

Plus particulièrement, la présente invention concerne un produit pharmaceutique comprenant, comme composants essentiels, de la poly(vinyl) pyrrolidone (PVP), de la carboxyméthylcellulose (CMC) et une substance active telle qu'un anesthésique.

La présente invention concerne également un emballage contenant ledit produit pharmaceutique, un procédé de préparation de ce produit et son utilisation comme médicament.

### ÉTAT DE LA TECHNIQUE ANTÉRIEURE

Concernant les soins dentaires, une anesthésie locale stoppe l'excitabilité des fibres nerveuses sensitives, de façon temporaire et réversible. Elle permet donc d'inhiber la douleur, pour un temps donné et est effectuée par une injection avec aiguille dans la zone adaptée en fonction de la dent - zone à traiter.

Toutefois, des méthodes ont été développées pour accompagner le patient vis-à-vis de son angoisse de l'injection mais aussi vis-à-vis de la douleur qu'elle peut générer reposant sur la pré-anesthésie ou anesthésie de surface. Il s'agit d'insensibiliser la muqueuse avant la piqûre, soit en utilisant un spray anesthésique ou un gel à l'endroit où l'injection va être faite, soit grâce à un spray cryogénique qui provoque un froid intense localisé pour bloquer les récepteurs sensitifs de la muqueuse.

Sont disponibles, en France, plusieurs produits pharmaceutiques à base d'anesthésiques de type amino-amide et notamment à base de lidocaïne, d'un sel de lidocaïne comme du chlorhydrate de lidocaïne (lidocaïne.HCl), ou d'un mélange de lidocaïne et de prilocaïne. On peut citer notamment les produits Dynexan^{®} 2%, Oraqix, Xylocontact, Xogel, Ziagel et Xylonor. Ces produits pharmaceutiques se présentent sous forme de gels, de crèmes buccales, et/ou de compresses imprégnées. Ils sont indiqués dans le cadre du traitement symptomatique des douleurs de la cavité buccale et de l'anesthésie locale de contact préalable à une anesthésie d'infiltration, à certaines explorations odonto-stomatologiques ou à certaines procédures dentaires telles que la taille et pose de couronnes et piliers de bridge, le détartrage juxta-gingival et sous-gingival et/ou le polissage radiculaire. Il convient de noter que la quantité maximale d'anesthésique dans ces produits est de 7,5% (Xylocontact). De plus, pour tous ces produits, l'anesthésie n'est que superficielle et de faible intensité et ne permet en aucun cas d'effectuer des soins douloureux qui concernent l'émail, la dentine ou les canaux.

Pour une anesthésie locale transdermique, des produits similaires, contenant souvent 2,5% de lidocaïne associée à 2,5% de prilocaïne, existent sous forme de crème ou de patchs. On peut citer les produits Emla 5% crème ou Emlapatch 5% et également d'autres produits très proches comme, les crèmes lidocaïne/prilocaïne 5%, Anesderm, Aguettant, Biogaran, Teva et Zentiva, certains de ces produits étant également déclinés en pansement. A nouveau, il s'agit d'une action très superficielle de courte durée et de faible intensité qui peut être faite pour limiter les inconvénients d'une injection ou d'un acte chirurgical cutané mineur.

Les produits et dispositifs utilisés dans le domaine de l'anesthésie présentent de nombreuses limitations.

Les dispositifs à aiguilles, utilisés pour injecter des anesthésiques génèrent des déchets biologiques tranchants qui doivent être éliminés par des circuits spécialisés. De plus, une partie non négligeable de la population souffre de phobie des aiguilles, et c'est un mode d'administration difficile à utiliser auprès de certains publics (enfants, personnes autistes, ...).

Pour les produits du type crème, gel ou patch « humide » à base de formulations anesthésiques, le temps d'induction de l'anesthésie est lent (temps de perméation de la drogue à travers la peau, la gencive ou la muqueuse), typiquement de 1h pour les patchs Emla par exemple, l'anesthésie résultante est une anesthésie légère de surface et est rarement suffisante pour des actes de soin courants, tels que les soins dentaires.

La recherche de nouveaux dispositifs et/ou nouvelles formulations pour favoriser une anesthésie rapide et profonde est donc un domaine actif. Ainsi, la revue « Recent Advances and Perspectives in topical oral anesthesia » de 2017 résume les pistes actuelles explorées dans le domaine de l'anesthésie dentaire **[1].**

Une première stratégie consiste à développer de nouvelles formulations d'anesthésiques, en les encapsulant dans des nano- ou micro-transporteurs (liposomes, particules polymériques, cyclodextrines...) pour prolonger leurs effets. Néanmoins, une telle stratégie ne favorise pas une anesthésie rapide puisque la substance active encapsulée dans le transporteur doit diffuser.

Une deuxième stratégie implique des méthodes physiques, telles que la cryothérapie, ou l'utilisation de courants électriques ou de micro-aiguilles non solubles couplées à l'utilisation d'une solution ou d'un gel d'anesthésique. Ces méthodes demandent l'utilisation d'un dispositif physique extérieur peu commode pour le praticien et le patient. Par exemple, pour l'utilisation de micro-aiguilles métalliques, céramiques ou polymériques non solubles, si la douleur du patient lors du geste d'anesthésie est réduite par rapport à une injection classique, il reste une partie piquante de dispositif à éliminer par le praticien, comme pour une injection classique.

De plus, pour les systèmes de micro-aiguilles solides avec revêtement par une formulation de polymère (séchée ou humide) comprenant des anesthésiques, le taux de charge en substance active est très faible (< 1 mg) car le volume disponible pour le chargement est très petit (surface des aiguilles). Un tel système a été décrit par Ma & Gill, 2014 **[2].**

Des micro-aiguilles polymériques non solubles, couplées à un patch réservoir de substance active en face arrière peuvent permettre d'embarquer une quantité importante de substance active. Ainsi le groupe de Kang a développé des patchs transdermiques à partir de micro-aiguilles polymériques non solubles en poly(éthylène glycol) di-acrylate réticulé par la lumière ; la phase polymère peut comporter jusque 21% en masse de lidocaïne, soit un contenu total de 86 mg de lidocaïne/patch. La diffusion de la substance active hors du patch est lente, prenant plusieurs heures (environ 8 mg relargués en 2h en solution) **[3].**

Une troisième stratégie consiste à développer de nouvelles formulations galéniques des anesthésiques, i.e. des films ou hydrogels bio-adhésifs complètement solubles. Ces formulations sont prometteuses en termes d'adhésivité, de facilité d'utilisation pour le praticien (sans résidus) et de compliance pour le patient. Néanmoins, la vitesse de délivrance de l'anesthésique est plutôt longue (1h ou plus) et la quantité de substance active embarquée dans les produits puis délivrée est relativement faible, souvent moins de 20% en poids par rapport à la phase polymérique. A noter que Sharmin *et al,* 2011 ont décrit un comprimé bicouche du type patch dans lequel la couche contenant la lipocaïne comprend un polymère polyacrylate réticulé du type carbopol, éventuellement associé à de l'hydroxypropyl-méthylcellulose (HPMC) **[4].** Cette couche comprend au plus 39% de lipocaïne (50 mg de lipocaïne et 80 mg de carbopol 934P). Les patchs ainsi décrits permettent une libération de 50% de la lipocaïne après 6h d'application.

Afin de renforcer l'adhésion du patch sur les tissus et de favoriser la diffusion de la substance active, des dispositifs à base de micro-aiguilles solubles ont été également proposés. Le taux de charge en anesthésiques dans ces systèmes est aujourd'hui modeste. Le plus élevé relevé dans la littérature est celui décrit par Ito *et al,* 2013 **[5],** avec un mélange 1/2 lidocaïne/chondroïtine sulfate pour la phase polymérique soluble formant les pointes des micro-aiguilles, additionnée d'une face arrière composée de 1/1 lidocaïne/acétate de cellulose, l'acétate de cellulose étant insoluble (seules les micro-aiguilles du dispositif sont solubles). Les dispositifs sont fabriqués par compression des poudres légèrement humidifiées puis séchage, en 2 étapes (micro-aiguilles puis ajout de la face arrière). Au final, la quantité de lidocaïne embarquée par patch est de 8,5 mg pour une surface de 1 cm². La présence de la face arrière insoluble permet d'embarquer une plus grande quantité d'anesthésique, mais ralentit la cinétique de libération, qui prend plus d'1h au total.

La demande de brevet US 2003/220403 A1 propose des compositions, sous forme solide comme des comprimés ou des gélules, à base de modafinil. Les formulations décrites dans les exemples 1, 2, 4 et 5 de cette demande contiennent entre 3,1% et 10% de poly(vinyl) pyrrolidone (PVP) et entre 3,1% et 5% de croscarmellose sodique, en masse par rapport à la masse totale des formulations.

La demande internationale WO 98/17263 A1 propose un patch transdermique ou buccal à plusieurs couches dont une des couches comprend entre 1% et 70% et, d'agent médical, entre 5% et 80% d'un composé polyvinylique tel que de la PVP et entre 1% et 50% d'un plastifiant.

La demande internationale WO 89/10740 A1 concerne un patch anesthésique adapté pour une utilisation buccale qui comprend (i) un film comprenant une matrice polymère, hydrophile, biocompatible, chargée avec l'anesthésique, (ii) une membrane support, inerte et imperméable et éventuellement (iii) une couche anti-adhésive.

La demande de brevet US 2015/216796 décrit un réseau de micro-aiguilles revêtues par un médicament et utiles pour injecter ce dernier dans la peau.

Dans le domaine des patchs solubles pour la délivrance topique, transdermale, transgingivale ou transmucosale d'anesthésiques, il n'existe pas encore de produits faciles à préparer, à manipuler, à conserver et à appliquer, ayant un important taux en substance active et permettant une libération totale de cette dernière en moins d'une heure sans laisser de résidus notamment tranchants. Les inventeurs se sont donc fixés pour but de proposer de tels produits.

### EXPOSÉ DE L'INVENTION

La présente invention permet d'atteindre le but que se sont fixé les inventeurs puisque ces derniers ont mis au point un produit pharmaceutique comprenant une phase polymérique particulière permettant d'obtenir non seulement un fort taux de charge en anesthésique de type amino-amide, ce taux étant supérieur à 40% en masse par rapport à la masse totale du produit, mais aussi une vitesse de dissolution très rapide dans l'eau et donc sur une peau, un tissu ou une muqueuse humide.

Le produit pharmaceutique selon l'invention se présente sous forme d'un matériau d'aspect sec, souple et facilement manipulable avant application. En présence d'eau ou d'une solution aqueuse, il est apte à se gonfler d'eau ou de la solution aqueuse puis de se dissoudre totalement i.e. sans générer de résidus, libérant ainsi l'anesthésique qu'il contient. Le produit pharmaceutique selon l'invention peut être défini comme une composition pharmaceutique solide : les expressions « produit pharmaceutique » et « composition pharmaceutique solide » sont équivalentes et utilisables de façon interchangeable dans la présente.

La phase polymérique particulière, mise en oeuvre dans le cadre du produit pharmaceutique selon l'invention est constituée de poly(vinyl) pyrrolidone (PVP) et de carboxyméthylcellulose (CMC) ou un de ses sels. Cette phase polymérique peut être ajustée pour présenter (i) des propriétés de plasticité ajustables, ce qui permet de conférer de la souplesse au produit selon l'invention pour venir se conformer à la géométrie des zones sur lesquelles il est appliqué ; (ii) des propriétés d'hygroscopie ajustables, ce qui permet une modulation de la vitesse initiale de délivrance de l'anesthésique; et (iii) des propriétés d'aspect (couleur) ajustables, ce qui permet de disposer de patchs transparents, colorés ou d'aspect blanc, toujours homogènes, sans altération des propriétés mécaniques.

Par ailleurs, le produit pharmaceutique peut être, facilement et rapidement, préparé avec des formulations aqueuses exemptes de tout solvant organique et sans chauffage évitant, de fait, d'endommager les anesthésiques mis en oeuvre. Ce produit pharmaceutique peut être structuré de façon aisée, notamment grâce à la faible viscosité de la phase polymérique et ce, afin de former des produits solubles micro- ou nano-structurés, par exemple comportant des micro-aiguilles sur la face apposée à la peau, aux tissus et aux muqueuses. Ces micro- ou nano-structurations participent, tout comme la nature de la phase polymérique, à l'adhésion du produit pharmaceutique selon l'invention au niveau de la peau, des tissus et des muqueuses. Le produit pharmaceutique selon l'invention peut être défini comme un dispositif pharmaceutique : les expressions « produit pharmaceutique » et « dispositif pharmaceutique » sont équivalentes et utilisables de façon interchangeable dans la présente.

De plus, le produit pharmaceutique selon l'invention peut être stocké, de façon stable et prolongée, à température ambiante, après packaging sous vide.

Même si les deux composés essentiels de la phase polymérique mise en oeuvre dans le produit pharmaceutique selon l'invention sont des polymères biocompatibles bien connus de l'homme du métier, il n'était du tout évident pour l'homme du métier que leur sélection parmi les nombreux polymères biocompatibles permettrait d'obtenir un produit pharmaceutique avec de telles propriétés.

Enfin, la présente invention est remarquable car elle s'applique non seulement au domaine des anesthésiques mais aussi, de façon plus générale, à toute substance active dans le domaine médical.

La présente invention concerne donc un produit pharmaceutique se présentant sous forme d'un film, d'un disque, d'une pastille, ou d'un patch pour une application sur la peau, un tissu ou une muqueuse comprenant :
- entre 40% et 65% en masse d'au moins une substance active ;
- entre 25% et 53% en masse de poly(vinyl) pyrrolidone (PVP) ; et
- entre 5% et 30% en masse de carboxyméthylcellulose (CMC) ou d'un de ses sels ;
les % en masse étant exprimés par rapport à la masse sèche totale dudit produit.

Dans le produit pharmaceutique selon l'invention, la substance active est dispersée, de façon homogène, dans la phase polymérique comprenant la PVP et la CMC. Comme déjà évoqué, le produit pharmaceutique selon la présente invention présente un aspect sec. En effet, il comprend au plus 5% en masse d'eau, notamment au plus 4% en masse et, en particulier, au plus 3% en masse par rapport à sa masse totale.

La ou les substance(s) active(s) susceptible(s) d'être mise en oeuvre dans le produit pharmaceutique selon l'invention peut(vent) être sélectionnée(s) parmi les principes actifs classiquement utilisés en pharmacothérapie et notamment dans les familles pharmacothérapeutiques suivantes : allergologie, anesthésie/ réanimation, chirurgie et notamment la cicatrisation, cancérologie et hématologie, cardiologie et angiologie, contraception et interruption de grossesse, dermatologie, endocrinologie, gastro-entéro-hépathologie, gynécologie et obstétrique, immunologie et médicaments de la transplantation, infectiologie et parasitologie, métabolisme, diabète et nutrition, neurologie/ psychiatrie, ophtalmologie, oto-rhino-laryngologie, pneumologie, rhumatologie, stomatologie, toxicologie, urologie/ néphrologie, ainsi que parmi les antalgiques/ anti-pyrétiques et antispasmodiques, anti-inflammatoires, les produits de diagnostic, les hémostatiques et les produits de traitement du sang et dérivés.

Notamment, la ou les substance(s) active(s) peut(vent) être sélectionnée(s) dans le groupe constitué par les principes actifs utiles ou utilisés pour l'anesthésie et/ou pour favoriser ou améliorer la cicatrisation. En effet, Baek *et al,* 2020 ont proposé des matériaux polymériques, notamment structurés en fibres comportant un anesthésique comme la lidocaïne pour des applications post-chirurgicales (réalisation de sutures ou apposition sur les tissus) et pour limiter l'adhésion et la formation de points focaux **[6].**

Avantageusement, la ou les substance(s) active(s) peut(vent) être sélectionnée(s) dans le groupe constitué par les anesthésiques du type amino-amide. Des exemples d'anesthésique de type amino-amide utilisables dans le cadre de la présente invention sont l'articaïne, la lidocaïne, la lévobupivacaïne, la mépivacaïne, la prilocaïne, la ropivacaïne et leurs sels pharmaceutiquement acceptables. En particulier, la ou les substance(s) active(s) peut(vent) être sélectionnée(s) dans le groupe constitué par la lidocaïne, un sel de lidocaïne comme du chlorhydrate de lidocaïne, un mélange de lidocaïne ou d'un sel de lidocaïne et de prilocaïne ou d'un sel de prilocaïne, l'articaïne et un sel d'articaïne.

Dans le produit pharmaceutique selon l'invention, la ou les substance(s) active(s) sont présentes en une quantité comprise entre 40% et 65% en masse, notamment entre 44% et 65% en masse, et, en particulier, entre 45% et 65% en masse par rapport à la masse sèche totale du produit.

La PVP mise en oeuvre dans le produit pharmaceutique selon l'invention est un polymère répondant à la formule générale (C₆H₉NO)ₙ. Typiquement, la PVP mise en oeuvre dans l'invention présente une masse moléculaire supérieure à 100 000 g/mol, notamment supérieure à 500 000 g/mol et, en particulier, comprise entre 1000 000 et 1500 000 g/mol.

Dans le produit pharmaceutique selon l'invention, la PVP est présente en une quantité comprise entre 25% et 53% en masse par rapport à la masse sèche totale du produit.

La CMC mise en oeuvre dans le produit pharmaceutique selon l'invention éventuellement sous forme d'un sel comme un sel de sodium ou un sel de potassium est un polymère répondant à la formule générale : 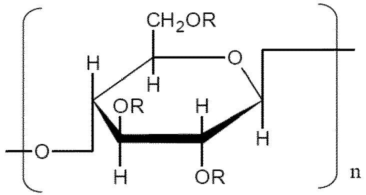 où R représente H ou CH₂COOX avec X = H, Na ou K.

La CMC mise en oeuvre dans le produit pharmaceutique selon l'invention, également connue sous l'appellation « carmellose », a pour numéro CAS 9000-11-7. Elle est différente de la « croscarmellose » qui est une carboxyméthylcellulose réticulée.

Typiquement, la CMC mise en oeuvre dans le produit pharmaceutique selon l'invention éventuellement sous forme d'un sel comme un sel de sodium ou un sel de potassium présente une masse moléculaire comprise entre 50 000 g/mol et 1 000 000 g/mol, notamment entre 60 000 g/mol et 500 000 g/mol et, en particulier, entre 70 000 g/mol et 300 000 g/mol.

Avantageusement, la CMC mise en oeuvre dans le produit pharmaceutique selon l'invention éventuellement sous forme d'un sel comme un sel de sodium ou un sel de potassium présente un degré de substitution compris entre 0,5 et 1 et notamment de l'ordre de 0,7 (i.e. 0,7 ± 0,1).

Dans le produit pharmaceutique selon l'invention, la CMC ou le sel de CMC comme un sel de sodium ou un sel de potassium de CMC est présent(e) en une quantité comprise entre 5% et 30% en masse par rapport à la masse sèche totale du produit.

Le produit pharmaceutique selon la présente invention peut éventuellement comprendre un ou plusieurs additifs pouvant moduler ses propriétés. Ainsi, le produit pharmaceutique selon la présente invention peut comprendre au plus 15% en masse par rapport à la masse sèche totale du produit, d'un ou plusieurs additif(s) choisi(s) parmi les sels organiques, les sels inorganiques, les correcteurs d'acidité, les plastifiants, les agents humectants et les correcteurs de couleur.

En d'autres termes, lorsque le produit pharmaceutique comprend plusieurs additifs tels que précédemment définis, la masse totale de ces additifs est inférieure ou égale à 15% par rapport à la masse sèche totale du produit.

Le ou les sel(s) organique(s) ou inorganique(s) que peut comprendre le produit pharmaceutique selon l'invention est/sont notamment utilisé(s) pour rendre la composition de la phase polymérique contenant la ou les substance(s) active(s) la plus proche possible du milieu physiologique (peau, tissus ou muqueuses), une fois à l'état hydraté.

Typiquement, le ou les sel(s) organique(s) ou inorganique(s) que peut comprendre le produit pharmaceutique selon la présente invention est/sont choisi(s) dans le groupe constitué par le chlorure de sodium (NaCl), le chlorure de potassium (KCI), le bicarbonate de sodium (NaHCO₃), le phosphate de sodium (Na₃PO₄), l'hydrogénophosphate de sodium (Na₂HPO₄), le dihydrogénophosphate de sodium (NaH₂PO₄), l'hydrogénophosphate de potassium (K₂HPO₄), le dihydrogénophosphate de potassium (KH₂PO₄), l'acide chlorhydrique (HCl), le chlorure de magnésium (MgCl₂), le chlorure de calcium (CaCl₂), le sulfate de sodium (Na₂SO₄), l'hydroxyde de sodium (NaOH), l'hydroxyde de potassium (KOH), l'acétate de sodium (CH₃COONa), le tartrate de sodium (Na₂C₄H₄O₆), le lactate de sodium (C(OH)(CH₃)COONa), le pyruvate de sodium (CH₃C(O)COONa), le citrate de sodium (Na₃C₆H₅O₇) et l'acide citrique (C₆H₈O₇).

Avantageusement, lorsque le produit pharmaceutique selon la présente invention comprend un ou plusieurs sel(s) organique(s) ou inorganique(s), la masse de ce ou de ces sel(s) est comprise entre 0,1% et 5%, notamment entre 0,2 et 2% par rapport à la masse sèche totale du produit.

Le ou les correcteur(s) d'acidité que peut comprendre le produit pharmaceutique selon l'invention est/sont notamment utilisé(s) pour corriger le pH de la phase polymérique contenant la ou les substance(s) active(s) notamment pour lui conférer des propriétés de flexibilité ajustables afin de se conformer au mieux à la forme de la peau, des tissus ou des muqueuses, et de mieux y adhérer.

Typiquement, le ou les correcteur(s) d'acidité que peut comprendre le produit pharmaceutique selon la présente invention est/sont choisi(s) dans le groupe constitué par l'acide acétique, l'acide chlorhydrique, l'acide citrique, l'acide malique, l'acide tartrique, l'acide métatartrique, l'acide lactique, l'acide phosphorique, l'acide adipique, l'acide fumarique, l'hydrazine, le carbonate de sodium, le carbonate de potassium, l'hydrogénocarbonate de sodium, l'hydrogénocarbonate de potassium, le pyrophosphate de sodium, le pyrophosphate de potassium, le citrate de sodium, le citrate de potassium, le gluconate de sodium et le gluconate de potassium. Dans un mode de réalisation particulier, le correcteur d'acidité que peut comprendre le produit pharmaceutique selon la présente invention est de l'acide acétique.

Avantageusement, lorsque le produit pharmaceutique selon la présente invention comprend un ou plusieurs correcteur(s) d'acidité, la masse de ce ou de ces correcteur(s) d'acidité est comprise entre 1% et 15%, notamment entre 3% et 10% par rapport à la masse sèche totale du produit.

Le ou les plastifiant(s) que peut comprendre le produit pharmaceutique selon l'invention est/sont notamment utilisé(s) pour conférer au produit des propriétés de flexibilité ajustables afin de se conformer au mieux à la forme de la peau, des tissus ou des muqueuses, et de mieux y adhérer.

Typiquement, le ou les plastifiant(s) que peut comprendre le produit pharmaceutique selon la présente invention est/sont choisi(s) dans le groupe constitué par les glycols présentant une masse moléculaire comprise entre 100 g/mol et 5 000 g/mol tels que le polyéthylène glycol (PEG) et le propylène glycol ; la lécithine ; l'acide citrique ; des huiles végétales comme de l'huile d'olive ou de l'huile de ricin ; des esters d'acide gras tels que le myristate d'isopropyle, le laurate d'hexyle, le sébacate de diéthyle, le sébacate de diisopropyle et le sébacate de dibutyle ; des amines telles que la monoéthanolamine, la diéthanolamine, la triéthanolamine, la monisopropanolamine et la triéthylenetetramine et des tensioactifs tels que des tensioactifs non-ioniques. A titre d'exemples de tensioactifs non-ioniques utilisables comme plastifiants dans le cadre de la présente invention, on peut citer les alcools polyéthoxylés, les phénols polyéthoxylés, les oléates, les laurates et leurs mélanges. A titre d'exemples non limitatifs de tensioactifs non-ioniques commerciaux, on peut citer les Triton X tels que le Triton X-100 ; les Brij tels que Brij-30 ; les Igepal CO tels que le Igepal CO-720 ; les Tween tels que le Tween 20 ; les Span tels que le Span 85.

Avantageusement, lorsque le produit pharmaceutique selon la présente invention comprend un ou plusieurs plastifiant(s), la masse de ce ou de ces plastifiant(s) est comprise entre 1% et 15%, notamment entre 3% et 10% par rapport à la masse sèche totale du produit.

Le ou les agent(s) humectant(s) que peut comprendre le produit pharmaceutique selon l'invention est/sont notamment utilisé(s) pour conférer au produit des propriétés hygroscopiques rendant encore plus rapide sa réhydratation lors de son application sur la peau, les tissus ou les muqueuses. Les agents humectants sont également désignés, dans la littérature, comme des agents hygroscopiques.

Typiquement, le ou les agent(s) humectant(s) que peut comprendre le produit pharmaceutique selon la présente invention est/sont choisi(s) dans le groupe constitué par le glycérol, les lactates et l'acide lactique, les monosaccharides tels que les hexoses et les pentoses, les polysaccharides hydrosolubles, le miel, le chlorure de sodium, le carbonate de calcium et le chlorure de calcium.

Avantageusement, lorsque le produit pharmaceutique selon la présente invention comprend un ou plusieurs plastifiant(s), la masse de ce ou de ces plastifiant(s) est comprise entre 0,1% et 15% par rapport à la masse sèche totale du produit.

Le ou les correcteur(s) de couleur que peut comprendre le produit pharmaceutique selon l'invention est/sont notamment utilisé(s) pour conférer au produit des propriétés d'aspect modulables, par exemple, passer d'un produit transparent incolore à un produit coloré transparent ou opaque. De plus, la présence de correcteurs de couleur à la surface de la peau, d'un tissu ou d'une muqueuse, une fois le produit pharmaceutique selon l'invention dissous, permet de visualiser l'emplacement de ce premier produit et de choisir le même emplacement ou, au contraire, un emplacement différent pour y appliquer un autre produit tel qu'un produit selon l'invention.

Typiquement, le ou les correcteur(s) de couleur que peut comprendre le produit pharmaceutique selon la présente invention est/sont choisi(s) dans le groupe constitué par les colorants tels que les colorants alimentaires ; du polyéthylène glycol de poids moléculaire compris entre 1000 g/mol et 10 000 000 g/mol ; et des charges minérales. Il est possible de combiner différents correcteurs de couleur entre eux pour obtenir l'aspect désiré pour le produit pharmaceutique selon l'invention.

Avantageusement, lorsque le produit pharmaceutique selon la présente invention comprend un ou plusieurs correcteur(s) de couleur, la masse de ce ou de ces correcteur(s) de couleur est comprise entre 0,01% et 15%, notamment entre 0,05% et 10% par rapport à la masse sèche totale du produit.

Le produit pharmaceutique selon la présente invention peut présenter une forme et une taille quelconques. Ces dernières pourront être choisies en fonction de l'utilisation envisagée pour le produit et notamment la morphologie de la zone sur laquelle le produit pharmaceutique selon l'invention doit être appliqué. Il se présente sous forme d'un film, d'un disque, d'une pastille ou d'un patch. Avantageusement, ce produit présente une masse par unité de surface comprise entre 10 mg/cm² et 1 g/cm² et notamment entre 25 mg/cm² et 500 mg/cm².

Par ailleurs, la surface du produit pharmaceutique selon la présente invention devant être apposée sur la peau, un tissu ou une muqueuse peut être lisse ou structurée. En effet, la nature de la phase polymérique mise en oeuvre lors de la préparation du produit et notamment sa faible viscosité permettent une structuration de sa surface qui peut être utile non seulement pour son adhésion mais aussi pour perforer la peau, un tissu ou une muqueuse et permettre l'administration transcutanée, transdermique, transgingivale et/ou transmucosale de la ou des substance(s) active(s) qu'il contient.

Dans un mode de réalisation particulier, la surface du produit pharmaceutique selon la présente invention devant être apposée ou appliquée sur la peau, un tissu ou une muqueuse présente des micro-structurations ou des nano-structurations. A titre d'exemples de telles structurations, on peut citer des micro-aiguilles capables de pénétrer dans les tissus comme une gencive, de la peau ou une muqueuse, sur une profondeur de 100 à 2000 µm, notamment de 200 à 1500 µm et, en particulier, de 300 à 1200 µm. Dans ce mode de réalisation particulier, la surface du produit pharmaceutique selon la présente invention devant être apposée ou appliquée sur la peau, un tissu ou une muqueuse présente des micro-aiguilles dont la hauteur moyenne est comprise entre 150 et 3000 µm, notamment entre 250 et 2000 µm et, en particulier, entre 300 et 1500 µm. Typiquement, la largeur moyenne à la base de ces micro-aiguilles est comprise entre 50 et 1000 µm, notamment entre 100 et 800 µm et, en particulier, entre 200 et 600 µm et/ou l'espace moyen inter-aiguilles est compris entre 150 et 3000 µm, notamment entre 250 et 2000 µm et, en particulier, entre 300 et 1500 µm.

De plus, le produit pharmaceutique selon la présente invention peut présenter un ou plusieurs élément(s) additionnel(s) utile(s) pour le manipuler et/ou pour le renforcer. A titre d'exemples particuliers de tels éléments, on peut citer un film plastique ou une pièce solide plastique notamment placé(e) sur la surface opposée à la surface devant être apposée ou appliquée sur la zone à traiter (peau, tissu ou muqueuse), un textile tissé ou un textile non tissé. Certains de ces éléments peuvent être soit ajoutés au ou placés sur le produit pharmaceutique, une fois ce dernier fabriqué (cas du film plastique ou de la pièce solide plastique), soit inclus au sein du produit (cas du textile tissé ou du textile non tissé). Par exemple, on peut envisager une pièce solide plastique avec une géométrie permettant de la tenir en face arrière entre pouce et index ainsi que le produit pharmaceutique selon l'invention, cette pièce étant, par la suite, désolidarisée du produit lors de la dissolution de celui-ci.

Le produit pharmaceutique selon la présente invention peut être stocké de façon prolongée à température ambiante, sous vide ou sous pression inférieure à la pression atmosphérique (pression négative). Ce stockage peut être réalisé dans un emballage du type sac plastique. Aussi, la présente invention concerne également un emballage dans lequel est stocké, sous vide ou sous inférieure à la pression atmosphérique (pression négative), un produit pharmaceutique tel que précédemment défini.

La présente invention concerne, en outre, un procédé de préparation d'un produit pharmaceutique tel que précédemment défini. Ce procédé comprend les étapes consistant à :
a) préparer une solution aqueuse contenant entre 40% et 65% en masse d'au moins une substance active, entre 25% et 53% en masse de poly(vinyl) pyrrolidone (PVP) et entre 5% et 30% en masse de carboxyméthylcellulose (CMC) ou d'un de ses sels, les % en masse étant exprimés par rapport à la masse sèche totale de ladite solution ;
b) appliquer ladite solution préparée à l'étape a) sur un support et
c) sécher ladite solution moyennant quoi un produit pharmaceutique est obtenu.

Tout ce qui a été précédemment décrit pour le produit pharmaceutique en termes de composants et additifs s'applique *mutatis mutandis* à la solution aqueuse préparée lors de l'étape a).

L'étape a) du procédé selon l'invention consiste à mélanger ensemble les différents composants de la solution, à savoir une ou des substance(s) active(s), de la PVP et de la CMC ou un de ses sels et éventuellement des additifs tels que précédemment décrits, dans un solvant aqueux de façon à obtenir une solution aqueuse dans laquelle sont dissous une ou plusieurs substance(s) active(s), de la PVP, de la CMC ou un de ses sels et éventuellement un ou plusieurs additif(s). Les composants et éventuels additifs peuvent être ajoutés en bloc, par groupe ou les uns après les autres.

Dans un mode de réalisation particulier, l'étape a) consiste à :
- ajouter à un solvant aqueux comme de l'eau ou de l'eau distillée, éventuellement additionné d'un ou plusieurs sel(s) organique(s) ou inorganique(s), envisagé(s) comme éventuel(s) additif(s), une ou des substance(s) active(s) puis mélanger l'ensemble, sous agitation notamment générée par un vortex de façon à obtenir une solution aqueuse contenant une ou des substance(s) active(s) ;
- ajouter à la solution aqueuse contenant une ou des substance(s) active(s) ainsi obtenue, de la PVP puis mélanger l'ensemble, sous agitation notamment générée par un vortex de façon à obtenir une solution aqueuse contenant une ou des substance(s) active(s) et de la PVP ;
- ajouter à la solution aqueuse contenant une ou des substance(s) active(s) et de la PVP ainsi obtenue, de la CMC ou un de ses sels puis mélanger l'ensemble, sous agitation notamment générée par un vortex de façon à obtenir une solution aqueuse contenant une ou des substance(s) active(s), de la PVP et de la CMC ou un de ses sels ;
- éventuellement ajouter à la solution aqueuse contenant une ou des substance(s) active(s), de la PVP et de la CMC ou un de ses sels ainsi obtenue, un ou plusieurs additif(s) puis mélanger l'ensemble, éventuellement sous agitation notamment générée par un vortex de façon à obtenir une solution aqueuse contenant une ou des substance(s) active(s), de la PVP, de la CMC ou un de ses sels et un ou plusieurs additif(s).

Il est possible de soumettre la solution aqueuse obtenue à l'issue de l'étape a) à une sonication afin d'éliminer, avant son dépôt sur le support, les bulles qu'elle pourrait contenir.

Typiquement, l'étape a) du procédé selon l'invention est réalisée à une température inférieure à 35°C, notamment à une température comprise entre 15°C et 30°C et, en particulier, à température ambiante (i.e. 23°C ± 5°C). La durée de l'étape a) est inférieure à 1h et notamment comprise entre 10 min et 45 min.

Le support sur lequel est déposée, lors de l'étape b), la solution aqueuse préparée lors de l'étape a) aura une forme et une géométrie, adaptées pour conférer, au produit pharmaceutique obtenu après séchage de la solution aqueuse, une forme donnée et notamment une forme micro- ou nano-structurée.

A cet effet, le support mis en oeuvre lors de l'étape b) peut être un moule et notamment un moule en silicone. Un tel moule en silicone peut être facilement obtenu par différentes méthodes connues de l'homme du métier. Ainsi, l'étape b) du procédé selon l'invention consiste à remplir ce moule avec la solution aqueuse préparée lors de l'étape a). Le remplissage du moule peut être effectué en appliquant un vide compris entre 50 et 500 mbar et notamment de l'ordre de 100 mbar (i.e. 100 mbar ± 10 mbar) par le dessous du moule. Une telle étape b) présente plusieurs avantages notamment par rapport à une méthode classique de dépôt par centrifugation : elle est transposable à l'échelle industrielle et compatible avec un procédé collectif de fabrication.

Il est possible de déposer, sur le support, préalablement à l'application de la solution aqueuse i.e. préalablement à l'étape b), un des éléments précédemment envisagés comme un textile tissé ou un textile non tissé de façon à ce que ce dernier soit pris/piégé dans le produit pharmaceutique obtenu en fin de procédé.

Typiquement, l'étape b) du procédé selon l'invention est réalisée à une température inférieure à 35°C, notamment à une température comprise entre 15°C et 30°C et, en particulier, à température ambiante (i.e. 23°C ± 5°C). La durée de l'étape b) est inférieure à 2h et notamment comprise entre 30 min et 90 min.

L'étape c) du procédé selon l'invention consiste à soumettre le support sur lequel a été appliquée la solution aqueuse préparée à l'étape a) à des conditions permettant le séchage de cette solution et ainsi d'obtenir un produit pharmaceutique selon la présente invention.

Typiquement le séchage lors de l'étape c) est effectué sous vide et à une température comprise entre la température ambiante et 60°C. La durée de l'étape c) sera choisie en fonction, d'une part, de la masse de la solution aqueuse déposée et, d'autre part, de la quantité d'eau résiduelle recherchée pour le produit pharmaceutique (quantité au moins inférieure à 5% en masse par rapport à la masse totale du produit). Avantageusement, cette durée est comprise entre 12h et 60h.

Dans un mode de réalisation particulier, l'étape c) du procédé peut comprendre au moins deux sous-étapes de séchages se distinguant, l'une de l'autre, par le vide appliqué et/ou par la température. Un exemple plus particulier de ce mode de réalisation consiste à soumettre la solution aqueuse appliquée sur le support du type moule à un premier séchage sous vide compris entre 150 mbar et 250 mbar et notamment de l'ordre de 200 mbar (i.e. 200 mbar ± 20 mbar) appliqué par le dessous du support à température ambiante pendant une durée comprise entre 10h et 15h et notamment de l'ordre de 12h (i.e. 12h ± 1h) ; puis à un deuxième séchage sous vide compris entre 10 mbar et 30 mbar et notamment de l'ordre de 20 mbar (i.e. 20 mbar ± 5 mbar) appliqué par le dessous du support à température ambiante pendant une durée comprise entre 10h et 15h et notamment de l'ordre de 12h (i.e. 12h ± 1h) et, enfin, à un troisième et dernier séchage sous vide compris entre 150 mbar et 250 mbar et notamment de l'ordre de 200 mbar (i.e. 200 mbar ± 20 mbar) à température comprise entre 40°C et 60°C et notamment de l'ordre de 50°C (i.e. 50°C ± 5°C) pendant une durée comprise entre 18h et 30h et notamment de l'ordre de 24h (i.e. 24h ± 2h). Un tel exemple d'étape c) est particulièrement adapté pour une solution aqueuse dont le volume appliqué sur le support est compris entre 0,05 ml et 3 ml et notamment entre 0,1 ml et 2 ml.

Une fois le produit pharmaceutique obtenu après l'étape c) de séchage, il est facilement récupérable à partir du support notamment par démoulage.

Suite à ce démoulage, il est possible d'associer, au produit, un des éléments envisagés, comme un film plastique ou une pièce solide plastique, de façon à ce que ce dernier soit appliqué sur une des surfaces et notamment sur la surface opposée à la surface mise en contact avec la peau, les tissus ou les muqueuses du produit pharmaceutique.

Le produit pharmaceutique ainsi préparé peut être conservé plusieurs semaines, i.e. au moins deux, au moins trois semaines ou encore au moins cinq semaines, à l'abri de l'humidité. Cette absence d'humidité peut être obtenue grâce à un sachet de desséchant et/ou sous vide et/ou sous gaz inerte comme de l'argon. Cette conservation peut être réalisée à une température comprise entre 4°C et 60°C. Dans certaines formes de mise en oeuvre, la conservation se fait à température ambiante ou à une température de l'ordre de 50°C (i.e. 50°C ± 5°C).

La présente invention concerne enfin un produit pharmaceutique tel que précédemment défini pour utilisation comme médicament et médicament à usage humain et/ou vétérinaire. Il est évident que la pathologie ou le trouble traité(e) ou prévenu(e) grâce au produit pharmaceutique selon la présente invention dépendra de la ou des substance(s) active(s) qu'il contient.

Dans une forme de mise en oeuvre particulière, ce produit pharmaceutique est utilisé comme agent anesthésique à appliquer sur la peau, un tissu ou une muqueuse ou pour favoriser ou améliorer la cicatrisation, notamment pour limiter l'adhésion tissulaire et la formation de points focaux au niveau des cicatrices. Il est évident que, pour une telle utilisation, le produit pharmaceutique contient au moins une substance active appartenant aux anesthésiques tels que précédemment définis.

En d'autres termes, la présente invention concerne un procédé pour anesthésier un animal ou un humain comprenant une étape consistant à apposer un produit pharmaceutique selon l'invention et contenant au moins une substance active appartenant aux anesthésiques tels que précédemment définis, sur la zone à anesthésier préalablement humidifiée, par exemple, en y appliquant de l'eau, de l'eau distillée ou du sérum physiologique moyennant quoi le produit pharmaceutique se réhydrate, gonfle et se dissout rapidement typiquement en une durée inférieure à 1h. Une fois le produit dissous, la ou les substance(s) active(s) qu'il contenait est libérée et peut diffuser au niveau et dans la zone à anesthésier. Cette dernière peut être de la peau, une gencive, un tissu ou encore une muqueuse.

En variante, la présente invention concerne un procédé pour favoriser ou améliorer la cicatrisation chez un animal ou un humain comprenant une étape consistant à apposer un produit pharmaceutique selon l'invention et contenant au moins une substance active appartenant aux anesthésiques tels que précédemment définis, sur la zone de cicatrisation préalablement humidifiée, par exemple, en y appliquant de l'eau, de l'eau distillée ou du sérum physiologique moyennant quoi le produit pharmaceutique se réhydrate, gonfle et se dissout rapidement typiquement en une durée inférieure à 1h. Une fois le produit dissous, la ou les substance(s) active(s) qu'il contenait est libérée et peut diffuser au niveau et dans la zone de cicatrisation. Cette dernière peut être de la peau, une gencive, un tissu ou encore une muqueuse.

D'autres caractéristiques et avantages de la présente invention apparaîtront encore à l'homme du métier à la lecture des exemples ci-dessous donnés à titre illustratif et non limitatif, en référence aux figures annexées.

### BRÈVE DESCRIPTION DES DESSINS

La Figure 1 présente une schématisation des étapes du procédé de préparation des dispositifs à micro-aiguilles par moulage.
La Figure 2 présente l'aspect de dispositifs structurés par des micro-aiguilles avec la géométrie A2, fabriqués à partir de différentes formulations.
La Figure 3 présente des photographies de peau humaine (sèche) après application des dispositifs à micro-aiguilles selon l'invention pendant quelques secondes.
La Figure 4 présente la photographie d'un dispositif à micro-aiguilles selon l'invention après 5 min d'apposition sur la gencive de porc.
La Figure 5 présente la diffusion de la lidocaïne dans des gels de gélatine/agar mimant les tissus après application d'un gel gingival (Xogel à 100 mg ou à 200 mg) ou d'un dispositif selon l'invention. (Passive représente les patchs de géométrie B2 préparés avec la formulation 4).
La Figure 6 présente la diffusion de la lidocaïne dans de la gencive de porc lors de deux expériences indépendantes (Passif représente les patchs de géométrie B2 préparés avec la formulation 4).

### EXPOSÉ DÉTAILLÉ DE MODES DE RÉALISATION PARTICULIERS

### Exemple 1 : Préparation de la phase polymérique

### Ingrédients

Les ingrédients utilisés dans les exemples ci-dessous sont décrits dans le Tableau 1 ci-après.

**Tableau 1**

| Produit | Masse Moléculaire (Da) | Degré de Substitution | Fournisseur | Référence |
|---|---|---|---|---|
| CMC | 95 000 | 0,7 | Ashland | Blanose^{™} CMC 7LP EP |
| PVP | 1 300 000 | | BASF | Kollidon^{®} K90F |
| Lidocaïne.HCl | | | Septodont | - |
| PEG | 20 000 | | Sigma Aldrich | Sigma Aldrich 81300 |

### Formulations de phases polymériques contenant des anesthésiques

Des exemples de formulations (Form.) de phases polymériques contenant des anesthésiques et correspondant à l'invention sont décrits dans le Tableau 2 ci-après.

**Tableau 2**

| **Ingrédient** | **Form. 1 (A)** | **Form. 2 (B)** | **Form. 4 NaCl** | **Form. 5 AA** | **Form. 6 PEG** |
|---|---|---|---|---|---|
| CMC | 17,2% (1170 mg) | 25,9% (225 mg) | 17,2% (1170 mg) | 16% (98 mg) | 16% (98 mg) |
| PVP | 34,5% (2340 mg) | 25,9% (225 mg) | 34,3% (2340 mg) | 32% (195 mg) | 32% (195 mg) |
| Lidocaïne.HCl | 48,3% (3276 mg) | 48,3% (420 mg) | 48% (3276 mg) | 44% (273 mg) | 44% (273 mg) |
| Acide acétique | - | - | | 8% (50 mg) | |
| PEG | - | - | | | 8% (50 mg) |
| NaCl* | - | - | 0,5% (34 mg) | | |
| *Eau* | *23400 mg (23,4 mL)* | *3000 mg (3 mL)* | *23400 mg (23,4 mL)* | *1950 mg (1,95 mL)* | |

Le chiffre indiqué dans le Tableau 2 correspond au % en masse de chaque ingrédient dans la phase polymérique avec anesthésique (masse totale sèche), le chiffre entre parenthèses correspond à la masse pesée pour la réalisation de la formulation. La dernière ligne indique la quantité d'eau rajoutée pour le procédé de fabrication et qui est ensuite éliminée par séchage. Le chlorure de sodium a été introduit dans la phase polymérique déjà dissous dans l'eau.

### Procédé

Pour obtenir la phase polymérique contenant un anesthésique, les étapes suivantes ont été réalisées :
- introduire l'anesthésique dans un flacon en verre, ajouter l'eau (ou la solution de NaCl) et vortexer 2 min à température ambiante pour bien dissoudre l'anesthésique ;
- ajouter la PVP et vortexer à nouveau 2 min ;
- ajouter la CMC et vortexer à nouveau 2 min ;
- ajouter les autres composants (acide acétique, PEG...) ; puis
- dissoudre complètement les polymères et éliminer les bulles en soniquant pendant environ 30 min, jusqu'à obtenir une solution claire et limpide.

### Exemple 2 : Moulage de la phase polymérique avec anesthésique de formulation 1 ou de la formulation 4 en dispositifs lisses ou structurés de différentes épaisseurs

La phase polymérique avec anesthésique de formulation 1 ou de formulation 4 a été utilisée pour mouler différents dispositifs en forme de pastilles lisses de paramètres géométriques suivants (dimensions générales du patch mesurées au pied à coulisse, dimension des aiguilles mesurées au microscope numérique Keyence VHX7000) :
- Dispositifs A1: Disques lisses de diamètre 18 mm, de hauteur 300 µm ;
- Dispositifs A2: Disques de diamètre 18 mm, de hauteur 300 µm (hors aiguilles, base du dispositif) et structurés sur la face supérieure par 137 micro-aiguilles de hauteur 700-730 µm, de largeur à la base 400 µm et d'espace inter-aiguilles 850 µm, disposées dans un cercle de 14 mm de diamètre au centre de la face supérieure du disque ;
- Dispositifs B1: Disques lisses de diamètre 18 mm, de hauteur 190 µm ;
- Dispositifs B2: Disques de diamètre 18 mm, de hauteur 190 µm (hors aiguilles, base du dispositif), et structurés sur la face supérieure par 137 micro-aiguilles de hauteur 700-730 µm, de largeur à la base 400 µm et d'espace inter-aiguilles 850 µm, disposées dans un cercle de 14 mm de diamètre au centre de la face supérieure du disque.

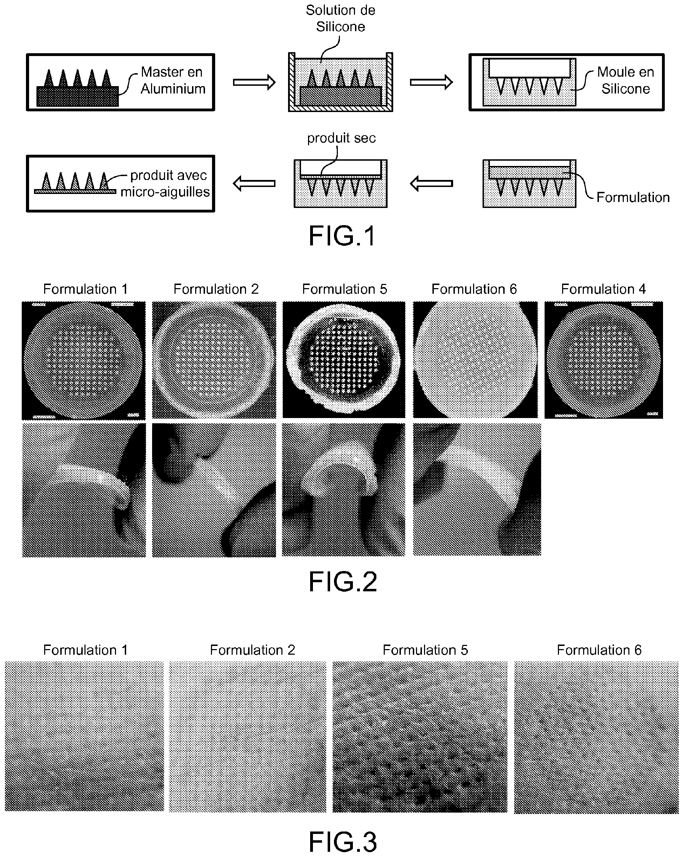

Les différentes étapes de préparation des dispositifs sont des étapes classiques pour l'homme du métier dans les procédés de moulage, et en particulier de moulage de dispositifs comprenant des micro-aiguilles. Ces étapes schématisées sur la Figure 1 sont les suivantes :
1) préparation de moules en silicone, négatifs des dispositifs polymériques souhaités : un master positif en aluminium a été usiné aux dimensions voulues. Ce positif est ensuite utilisé pour fabriquer le moule en silicone dans lequel sera coulée la phase polymérique avec un anesthésique. Nous avons utilisé des moules en silicone comportant 3 ou 9 cavités pour le moulage simultané de 3 ou 9 dispositifs.
2) versement de la phase polymérique avec anesthésique (formulation 1 ou formulation 4 dans les moules en silicone et remplissage complets des moules : le moulage est réalisé à température ambiante en versant dans chaque moule la quantité de polymère nécessaire, soit :
   - pour les dispositifs A1 et A2 : 650 µL de formulation 1 ou 4 ;
   - pour les dispositifs B1 et B2 : 425 µL de formulation 1 ou 4.
   Afin de remplir complètement la cavité (notamment dans le cas des dispositifs structurés par des micro-aiguilles), le dispositif est placé 1 h à température ambiante sous vide (100 mbar), le vide étant appliqué par le dessous du moule en silicone.
3) séchage de la phase polymérique avec anesthésique sous vide, avec les étapes suivantes :
   - 12 h, 200 mbar, température ambiante, vide appliqué par le dessous du moule en silicone ;
   - 12 h, 20 mbar, température ambiante, vide appliqué par le dessous du moule en silicone ; et
   - 24 h, 200 mbar, 50°C dans une étuve sous vide de marque ThermoFisher modèle Vacutherm pour compléter le séchage.
4) démoulage des dispositifs : Les dispositifs secs sont démoulés aisément grâce à la plasticité du moule en PDMS. La masse des dispositifs secs ainsi obtenus est de 151 mg pour les dispositifs A1 et A2 et de 99 mg pour les dispositifs B1 et B2.
5) packaging des dispositifs pour conservation longue durée : Les dispositifs séchés sont conservés au moins pendant plusieurs semaines à l'abri de l'humidité :
   - soit à 50°C dans une étuve avec un sachet de desséchant ;
   - soit disposés dans une boîte avec un desséchant sous argon, la boîte étant ensuite placée dans un sac en plastique et packagée sous vide jusqu'à utilisation.

### Exemple 3 : Réalisation et caractérisation de dispositifs micro-aiguilles de géométrie A2 avec les formulations 1 à 6

Des dispositifs micro-aiguilles (MN) de géométrie A2 ont été réalisés avec les formulations 1 à 6 suivant le protocole détaillé à l'exemple 2. Ces dispositifs ont ensuite été caractérisés de la façon suivante :
1) Leur aspect général : couleur et plasticité : Les photos des dispositifs de formulations 1, 2, 4, 5 et 6 sont présentés Figure 2.

Les dispositifs sont transparents (Formulations 1, 2, 4 et 5), mais peuvent être rendus opaques par ajout de PEG 20 kDa à une quantité de 8% (Formulation 6). De même l'ajout de colorants, combinés ou non au PEG, peut permettre de moduler leur aspect sans altérer leur fonctionnalité en termes de délivrance de l'anesthésique (voir ci-dessous).

Les dispositifs sont relativement souples et non cassants, ce qui permet de les conformer à la géométrie des tissus. L'ajout de correcteurs d'acidité jouant le rôle de plastifiant, tel l'acide acétique (Formulation 5), permet d'augmenter la flexibilité du patch sans pour autant altérer sa capacité à perforer les tissus (voir ci-dessous).

2) Leurs propriétés géométriques sont caractérisées par un microscope numérique Keyence VHX 7000 qui permet de mesurer notamment la hauteur des micro-aiguilles. Les résultats des mesures sont regroupés dans le Tableau 3.

**Tableau 3**

| **Paramètre** | **Form. 1** | **Form. 2** | **Form. 4** | **Form. 5** | **Form. 6** |
|---|---|---|---|---|---|
| Hauteur des micro-aiguilles | 705 ± 27 µm | 671 ± 30 µm | 703 ± 19 µm | 701 ± 29 µm | 721 ± 53 µm |

Une caractérisation plus poussée a été menée avec deux séries de 45 dispositifs issues des compositions des formulations 1 et 4. Dans ce cas, ont été mesurés de façon systématique :
- La hauteur des MN et le % par rapport au master aluminium correspondant ;
- Le volume des MN et le % par rapport au master aluminium correspondant ;
- La planéité du dispositif, estimée en mesurant la différence maximale (Zmax- Zmin) de hauteur d'un bord à l'autre du dispositif sur deux profils orthogonaux (12 mm par profil). Avec cette mesure, il est possible de calculer le pourcentage de la pente de la courbure pour les deux profils orthogonaux, comme % = 100 x (Zmax-Zmin) / Longueur, la longueur étant égale à 6 mm (la moitié du profil de 12 mm longueur) ;
- L'homogénéité des dispositifs, évaluée quantitativement par la moyenne et le CV (écart-type / moyenne) des niveaux de gris mesurés sur une image du dispositif.

Les résultats obtenus sont regroupés dans le Tableau 4. Ils soulignent la planéité des dispositifs obtenus (courbure de 2,0 ± 0.9 sur les 2 séries), ainsi que leur homogénéité (CV de 0,031 ± 0,011 sur les 2 séries).

**Tableau 4**

| **Formulation** | **Masse Dispositif** | **Hauteur MN** | | **Volume MN** | | **Courbure (%)** | **Homogénéité** | |
|---|---|---|---|---|---|---|---|---|
| | | **(µm)** | **% master aluminium** | **(nL)** | **% master aluminium** | | **Moyenne (niveau gris)** | **CV (niveau gris)** |
| **Form. 1** | **150,8** | **705,1** | **95,2** | **37,4** | **76,7** | **1,7** | **104,4** | **0,028** |
| *Ecart-type* | *4,4* | *26,7* | *3,1* | *3,4* | *4,8* | *0,6* | *5,4* | *0,011* |
| **Form. 4** | **150,6** | **703,4** | **94,7** | **38,7** | **78,3** | **2,3** | **99,4** | **0,034** |
| *Ecart-type* | *6,0* | *19,3* | *2,2* | *3,1* | *3,9* | *1,0* | *4,3* | *0,011* |
| **Moyenne Form. 1 et 4** | **150,7** | **704,2** | **94,99** | **38,0** | **77,5** | **2,0** | **101,9** | **0,031** |
| *Ecart-type* | *5,2* | *23,1* | *2,66* | *3,3* | *4,4* | *0,9* | *5,4* | *0,011* |

Ils démontrent également la fiabilité et la reproductibilité du procédé de fabrication : 72 dispositifs/90 (80%) ont présenté une masse de dispositif, une hauteur des MN, un volume des MN, un % de courbure, une homogénéité de couleur compris entre (moyenne - 2 écart-type) pour toutes les séries < valeur pour le dispositif < (moyenne + 2 écart-type) pour toutes les séries.

3) Leur caractérisation mécanique : les dispositifs sont fixés sur une sonde cylindrique en aluminium de 20 mm spécialement usinée par un biseau pour y fixer les dispositifs plans de 18 mm de diamètre. Cette sonde est adaptée au texturomètre TA.XT Plus (Swantech, Gennevillers, France) afin de pouvoir comprimer les dispositifs sur un plan en acier avec une force connue. La force appliquée sur l'ensemble du dispositif est de 49N, soit 0,355 N/micro-aiguille. La perte en hauteur des micro-aiguilles est mesurée au microscope numérique Keyence VHX 7000 et exprimée en % après la compression.

Les résultats sont résumés dans le Tableau 5 avec « H0 » représentant la hauteur des micro-aiguilles avant compression et « H après » la hauteur des micro-aiguilles après compression.

**Tableau 5**

| **Echantillon** | **H0 (µm)** | **H après (µm)** | **% diminution** |
|---|---|---|---|
| **Formulation 1** | | | |
| ME587-A1 | 675 ± 43 (11) | 599 ± 30 (11) | 11,2% |
| ME587-A2 | 671 ± 43 (11) | 643 ± 37 (11) | 4,1% |
| ME593-C2 | 728 ± 55 (11) | 677 ± 20 (11) | 7,0% |
| *Moyenne* | | | *7,4 ±3,6%* |

| **Formulation 1 Après 84 j de conservation** | | | |
|---|---|---|---|
| ME591-A1 | 723 ± 33 (11) | 655 ± 22 (11) | 9,4% |
| ME591-A2 | 672 ± 19 (11) | 652 ± 23 (11) | 3,1% |
| *Moyenne* | | | *6,3 ± 4,5%* |

| **Formulation 4** | | | |
|---|---|---|---|
| ME601-A1 | 721 ± 41 (11) | 676 ± 8 (11) | 6,3% |
| ME601-A2 | 673 ± 20 (11) | 635 ± 32 (11) | 5,7% |
| *Moyenne* | | | *6,0 ± 0,4%* |

| **Formulation 5** | | | |
|---|---|---|---|
| ME587-B3 | 718 ± 23 (5) | 695 ± 22 (5) | 3,2% |
| ME589-B1 | 667 ± 57 (5) | 614 ± 52 (5) | 8,0% |
| *Moyenne* | | | *5,6 ± 3,4%* |

| **Formulation 6** | | | |
|---|---|---|---|
| ME587-C3 | 776 ± 23 (5) | 758 ± 22 (5) | 2,3% |
| ME589-C1 | 716 ± 26 (5) | 659 ± 24 (5) | 10,1% |
| *Moyenne* | | | *6,2 ± 5,5%* |

4) Insertion dans le plasticlay : Les dispositifs MN ont ensuite été testés pour l'insertion dans du plasticlay, un matériau souple, bien que non humide, donc adapté pour effectuer des mesures mécaniques lors de l'insertion des dispositifs en évitant leur dissolution rapide. De plus, nous avons mesuré au texturomètre TA.XT Plus que la plasticlay présente un module de Young de 6 à 10 MPa, similaire à la peau humaine. Il représente donc un modèle intéressant et facile pour tester la perforation cutanée, découplée de la dissolution des dispositifs.

Les dispositifs sont insérés dans le plasticlay à l'aide du texturomètre TA.XT Plus avec une force de 25 N. La hauteur des MN avant/après insertion est mesurée au microscope numérique Keyence VHX 7000, ainsi que la profondeur des perforations dans le plasticlay. Les résultats sont regroupés dans le Tableau 6.

**Tableau 6**

| **Dispositif à MN** | **Hauteur des MN (µm)** | | **Profondeur des perforations (µm)** |
|---|---|---|---|
| | Avant insertion | Après insertion | |
| ME604-A2 (Form. 1) | 702 ± 32 | 677 ± 32 (-3,5 %) | 412 ± 58 |
| ME593-B1 (Form. 1) | 739 ± 32 | 746 ± 22 (+1,0 %) | 362 ± 45 |
| ME591-A3 (Form. 1) Après 84 j de conservation | 734 ± 24 | 737 ± 21 (+0,5 %) | 410 ± 61 |
| ME591-B1 (Form. 1) After 84 days storage | 705 ± 36 | 697 ± 42 (-1,2 %) | 369 ± 28 |
| ME601-B1 (Form. 4) | 683 ± 50 | 677 ± 57 (-0,9 %) | 313 ± 50 |
| ME601-B2 (Form. 4) | 716 ± 28 | 712 ± 33 (-0,5 %) | 364 ± 39 |
| MRM05-B1 (Form. 2) | 671 ± 30 | - | 412 |
| ME589-B2 (Form. 5) | 718 ± 33 | 718 ± 33 (-) | 302 |
| ME587-C3 (Form. 6) | 776 ± 23 | 719 ± 22 (-7,4 %) | 326 |

5) Insertion dans les tissus : Les dispositifs MN peuvent être insérés dans les tissus où ils créent des perforations, tel que montré Figure 3.

De plus, si la surface du tissu a été préalablement humidifiée (par exemple à l'aide d'un badigeon ou d'un tampon imbibés d'eau ou de sérum physiologique), le dispositif se met à prendre l'eau, gonfler, puis se dissoudre progressivement.

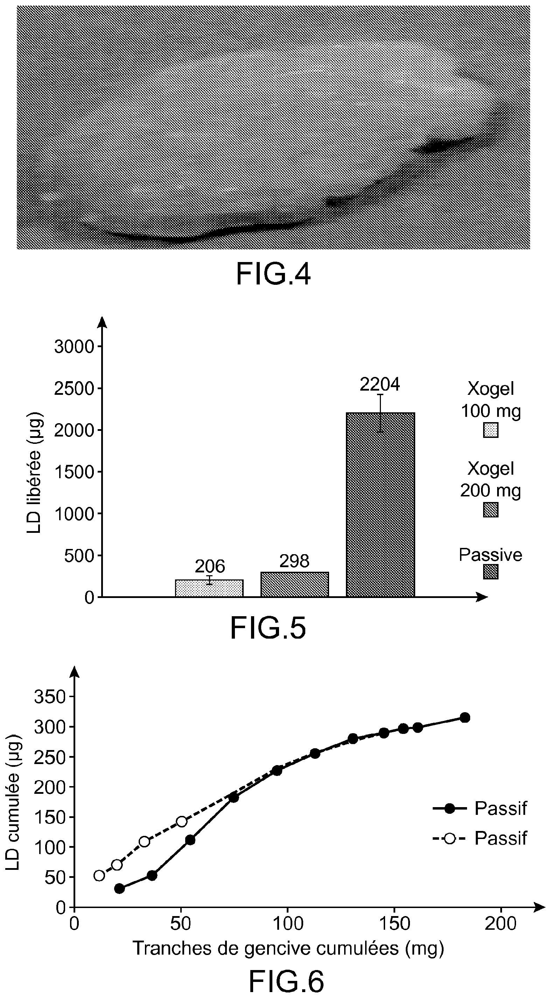

Ainsi, il a été observé, pour des dispositifs apposés sur la gencive de porc, une prise de poids de +25% après 3 min d'apposition et une prise de poids de +75% après 5 min d'apposition, alors que le dispositif a manifestement été en partie dissous (disparition de la structuration micro-aiguilles notamment, Figure 4).

6) Cinétique de dissolution des dispositifs *(in vitro) :* Les dispositifs MN de formulation 1 ou de formulation 4 sont introduits dans 5 ml d'eau sous agitation magnétique (350 tr/min) à température ambiante (20°C). Au temps voulu, 100 µL de la solution sont prélevés et remplacés par 100 µL d'eau. Les échantillons prélevés sont dilués 10 fois dans de l'eau (100 µL d'échantillon + 900 µL d'eau), filtrés sur des filtres Acrodisc ^{™} de 0,2 µm avant analyse UPLC.

Les paramètres d'analyse UPLC sont résumés dans le Tableau 7. Une courbe d'étalonnage est établie avec ces conditions UPLC en utilisant des solutions standard de lidocaïne.HCl dans la gamme 2-600 µg/mL préparées dans l'eau puis filtrées sur 0,22 µm. Elle permet de remonter à la quantité de lidocaïne dissoute au cours du temps lors de l'expérience de dissolution des dispositifs (Tableau 8). Dans cette expérience, l'erreur estimée sur les valeurs (pipetage, dissolution,...) est ± 2%. La dissolution des dispositifs se fait en 45 min, avec plus de 80% des dispositifs solubilisés à 20 min.

**Tableau 7**

| Système UPLC (pompe) | Waters AcquityH UPLC^{®}Class |
|---|---|
| UPLC detector | Détecteur Waters TUV fonctionnant à 265 nm |
| Colonne | Acquity UPLC^{®} BEH C18 1,7 µm, 2,1 × 50 mm |
| Eluant | 20 mM phosphate pH 8.0 / Méthanol, 40/60 |
| Débit | 0,3 mL/min |

**Tableau 8**

| Temps (min) | ME591-B2 (Form. 1) | ME591-B3 (Form. 1) | ME591-C3 (Form. 1) | ME601-B3 (Form. 4) | ME602-A2 (Form. 4) |
|---|---|---|---|---|---|
| 0 | 0,0 | 0,0 | 0,0 | 0,0 | 0,0 |
| 2 | 32,5 | 30,7 | 27,0 | 26,3 | 25,4 |
| 4 | 55,0 | 50,6 | 41,0 | 36,9 | 41,3 |
| 6 | 57,2 | 67,4 | 47,2 | 48,6 | 52,8 |
| 8 | 65,8 | 86,0 | 55,8 | 60,1 | 71,6 |
| 10 | 75,5 | | 65,3 | 72,6 | 85,4 |
| 15 | 77,2 | 88,1 | 82,9 | 88,6 | 94,4 |
| 20 | 88,8 | 96,9 | 84,4 | 96,8 | 98,3 |
| 30 | 97,8 | 98,8 | 92,7 | 99,1 | **100,0** |
| 45 | **100,0** | **100,0** | 99,2 | 99,3 | |
| 60 | | | **100,0** | **100,0** | |

Au bout de 2h de dissolution, on obtient la quantité totale d'anesthésique comprise dans le dispositif, tel qu'indiqué dans le Tableau 9. On retrouve le % massique attendu.

**Tableau 9**

| **Dispositif à MN** | **Masse Dispositif (mg)** | **Contenu LD (mg)** | **% LD dans phase polymérique** |
|---|---|---|---|
| ME591-B2 (Form. 1) | 132,5 | 65,0 | 49,0 |
| ME591-B3 (Form. 1) | 131,1 | 61,6 | 47,0 |
| ME591-C3 (Form. 1) | 130,0 | 59,1 | 45,5 |
| ME601-B3 (Form. 4) | 151,5 | 76,5 | 50,5 |
| ME602-A2 (Form. 4) | 154,6 | 76,7 | 49,6 |
| **Moyenne** | | | **48,3 ± 2,1** |

### Exemple 4 : Diffusion de la lidocaïne à partir de dispositifs préparés à partir de la formulation 4

La diffusion de la lidocaïne dans des gels de gélatine/agar et des modèles de tissu ou des tissus de gencives de porc *ex vivo* a été étudiée.

### Diffusion dans les gels de gélatine/agar

Les gels de gélatine-agar ont été préparés en dissolvant à température modérée, 60°C, 12 g de gélatine et 0,5 g d'agar dans une solution de NaCl 154 mM (37,5 mL) avec une agitation très douce afin d'éviter la formation de bulles. Cette solution a ensuite été soniquée jusqu'à disparition des bulles et 18 ml sont versés dans une boîte de Pétri de 8,8 cm de diamètre conduisant à un gel de 2,2 mm d'épaisseur.

Le dosage de la lidocaïne diffusée dans les gels de gélatine/agar après 15 min d'application des dispositifs sur le gel a été réalisé afin de quantifier la diffusion de l'anesthésique dans ce modèle de tissu. Sont comparés le dispositif avec micro-aiguilles (géométrie A2 détaillée à l'exemple 2, d'une masse de 150 mg, correspondant à 71 mg de lidocaïne-HCl) préparé avec la formulation 4 et une application du produit commercial Xogel fourni par la société Septodont.

Pour le test Xogel, une quantité contrôlée a été déposée sur le dessus du gel et correctement répartie sur toute la surface du gel. Les quantités déposées correspondent à des doses minimales et maximales selon la posologie indiquée pour le produit, à savoir 100 et 200 mg de gel, correspondant à 5 et 10 mg de lidocaïne. Après 15 min d'application des patchs ou du Xogel, l'excès de Xogel ou les restes du patch sont éliminés avec un nettoyage soigneux à la spatule.

Le gel de gélatine-agar a ensuite été congelé à -80°C puis découpé en tranches de 150 µm avec un cryomicrotome. Chaque tranche a été dissoute dans 1,2 ml d'acétonitrile/eau (10/90) avec une incubation de 5h à 37°C sous agitation continue à 500 tr/min. Après dissolution de chaque tranche de gel, le mélange est centrifugé et le surnageant est analysé par UPLC-ELSD dans les mêmes conditions que décrites précédemment.

Les résultats de la Figure 5 indiquent que la dose totale de lidocaïne délivrée en utilisant le Xogel est 10 fois inférieure à la dose délivrée par le patch.

### Diffusion dans la gencive de porc ex vivo

Les gencives de porc ont été coupées à l'aide d'un scalpel chirurgical à partir de têtes de porc fournies par un éleveur à la sortie de l'abattoir et congelées à -18°C jusqu'à utilisation.

Pour déterminer la diffusion de la lidocaïne dans les gencives de porc, les échantillons de tissus ont été rapidement réhydratés en les immergeant dans une solution saline physiologique, NaCl 154 mM, pendant 3 secondes et rapidement épongés sur papier absorbant non tissé.

Les dispositifs avec micro-aiguilles (géométrie A2 détaillée à l'exemple 2, d'une masse de 150 mg, correspondant à 71 mg de lidocaïne-HCl) préparés avec la formulation 4 sont ensuite appliqués sur le tissu où ils sont maintenus par une masse de 10 g, et ce pour une application de 15 min.

Pour déterminer la quantité de lidocaïne diffusée à l'intérieur du tissu, celui-ci est placé dans une boîte de Pétri ensuite remplie de milieu de congélation tissulaire (milieu de congélation tissulaire OCT, réf 2545932, TBS), puis congelé très rapidement dans l'azote liquide et coupé en tranches de 150 µm d'épaisseur avec le cryoMicrotome Leica (sur une épaisseur d'environ 2 mm). Puis, pour chaque tranche de 150 µm d'épaisseur, l'échantillon a été dissous avec 100 µl de CH₃CN à 10% (eau à 90%), puis incubé pendant 5h à 37°C et 300 tr/min pour l'extraction de la lidocaïne des tissus. Pour la partie de tissu restante non coupée au cryostat, 500 µl de la solution de CH₃CN à 10% (eau à 90%) ont été ajoutés puis incubés dans les mêmes conditions que ci-dessus. Après une centrifugation pendant 5 min à 2000 g, le surnageant a été filtré et injecté pour une analyse HPLC-UV.

Les résultats de la Figure 6 montrent que, dans deux expériences indépendantes, 300-350 µg de lidocaïne ont diffusé dans 150-200 mg de tissu en 15 min d'application du dispositif à micro-aiguilles.

### Références

**[1]** Franz-Montan et al, 2017, « Recent advances and perspectives in topical oral anesthesia », Expert. Opin. Drug Deliv., vol. 14, pages 673-684.
**[2]** Ma & Gill, 2014, « Coating solid dispersions on microneedles via a molten dip coating method: development and in vitro evaluation for transdermal delivery of a water insoluble drug », J. Pharm. Sci., vol. 103, pages 3621-3630.
**[3]** Kochlar et al, 2013, « Microneedle Integrated Transdermal Patch for Fast Onset and Sustained Delivery of Lidocaine », Mol. Pharmaceutics, vol. 10, pages 4272-4280.
**[4]** Sharmin et al, 2011, « Préparation and Characterization of Lidocaine Double Layer Buccal Tablet Using Mucoadhesive Carbopol® Polymers », J. Pharm. Sci., vol. 10, pages 29-34.
**[5]** Ito et al, 2013, « Dissolving microneedles to obtain rapid local anesthetic effect of lidocaine at skin tissue », J. Drug Targeting, vol. 21, pages 770-775.
**[6]** Baek et al, 2020, « Development of a Lidocaine-Loaded Alginate/CMC/PEO Electrospun Nanofiber Film and Application as an Anti-Adhesion Barrier », Polymers, Vol. 12, No. 618.

## Revendications

1. Produit pharmaceutique se présentant sous forme d'un film, d'un disque, d'une pastille, ou d'un patch pour une application sur la peau, un tissu ou une muqueuse comprenant :
- entre 40% et 65% en masse d'au moins une substance active ;
- entre 25% et 53% en masse de poly(vinyl) pyrrolidone (PVP) ; et
- entre 5% et 30% en masse de carboxyméthylcellulose (CMC) ou d'un de ses sels ;
les % en masse étant exprimés par rapport à la masse sèche totale dudit produit.

2. Produit pharmaceutique selon la revendication 1, **caractérisé en ce que** ladite ou lesdites substance(s) active(s) est/sont sélectionnée(s) dans le groupe constitué par les principes actifs utiles ou utilisés pour l'anesthésie et/ou pour favoriser ou améliorer la cicatrisation.

3. Produit pharmaceutique selon la revendication 1 ou 2, **caractérisé en ce que** ladite ou lesdites substance(s) active(s) est/sont sélectionnée(s) dans le groupe constitué par les anesthésiques du type amino-amide et, en particulier, dans le groupe constitué par la lidocaïne, un sel de lidocaïne, un mélange de lidocaïne ou d'un sel de lidocaïne et de prilocaïne ou d'un sel de prilocaïne, l'articaïne et un sel d'articaïne.

4. Produit pharmaceutique selon l'une quelconque des revendications 1 à 3, **caractérisé en ce que** ladite ou lesdites substance(s) active(s) sont présentes en une quantité comprise entre 45% et 65% en masse par rapport à la masse sèche totale dudit produit.

5. Produit pharmaceutique selon l'une quelconque des revendications 1 à 4, **caractérisé en ce qu'**il comprend au plus 15% en masse par rapport à sa masse sèche totale d'un ou plusieurs additif(s) choisi(s) parmi les sels organiques, les sels inorganiques, les correcteurs d'acidité, les plastifiants, les agents humectants et les correcteurs de couleur.

6. Produit pharmaceutique selon l'une quelconque des revendications 1 à 5, **caractérisé en ce que** la surface dudit produit devant être apposée ou appliquée sur la peau, un tissu ou une muqueuse présente des micro-aiguilles dont la hauteur moyenne est comprise entre 150 et 3000 µm.

7. Produit pharmaceutique selon l'une quelconque des revendications 1 à 6, **caractérisé en ce qu'**il présente un ou plusieurs élément(s) additionnel(s) choisi(s) dans le groupe constitué par un film plastique, une pièce solide plastique, un textile tissé ou un textile non tissé.

8. Emballage dans lequel est stocké, sous vide ou sous pression inférieure à la pression atmosphérique, un produit pharmaceutique tel que défini dans l'une quelconque des revendications 1 à 7.

9. Procédé de préparation d'un produit pharmaceutique tel que défini dans l'une quelconque des revendications 1 à 7, ledit procédé comprenant les étapes consistant à :
a) préparer une solution aqueuse contenant entre 40% et 65% en masse d'au moins une substance active, entre 25% et 53% en masse de poly(vinyl) pyrrolidone (PVP) et entre 5% et 30% en masse de carboxyméthylcellulose (CMC) ou d'un de ses sels, les % en masse étant exprimés par rapport à la masse sèche totale de ladite solution ;
b) appliquer ladite solution préparée à l'étape a) sur un support et
c) sécher ladite solution moyennant quoi un produit pharmaceutique est obtenu.

10. Procédé de préparation selon la revendication 9, **caractérisé en ce que** ledit support mis en oeuvre lors de l'étape b) est un moule et notamment un moule en silicone.

11. Procédé de préparation selon la revendication 10, **caractérisé en ce que** ladite étape b) consiste à remplir ledit moule avec la solution aqueuse préparée lors de l'étape a) et à appliquer un vide compris entre 50 et 500 mbar par le dessous du moule.

12. Produit pharmaceutique tel que défini dans l'une quelconque des revendications 1 à 7, pour utilisation comme médicament à usage humain et/ou vétérinaire.

13. Produit pharmaceutique tel que défini dans l'une quelconque des revendications 1 à 7, pour utilisation comme agent anesthésique à appliquer sur la peau, un tissu ou une muqueuse ou pour favoriser ou améliorer la cicatrisation, notamment pour limiter l'adhésion tissulaire et la formation de points focaux au niveau des cicatrices.

## Patentansprüche

1. Pharmazeutisches Produkt in Form eines Films, einer Scheibe, einer Tablette oder eines Pflasters zur Anwendung auf der Haut, einem Gewebe oder einer Schleimhaut, umfassend:
- zwischen 40 Massen-% und 65 Massen-% mindestens einer Wirksubstanz;
- zwischen 25 Massen-% und 53 Massen-% Poly(vinyl)pyrrolidon (PVP); und
- zwischen 5 Massen-% und 30 Massen-% Carboxymethylcellulose (CMC) oder eines ihrer Salze;
wobei die Massen-% in Bezug auf die gesamte Trockenmasse des Produkts ausgedrückt werden.

2. Pharmazeutisches Produkt nach Anspruch 1, **dadurch gekennzeichnet, dass** die Wirksubstanz(en) aus der Gruppe ausgelesen ist(sind), die aus Wirkstoffen besteht, die zur Anästhesie und/oder zur Unterstützung oder Verbesserung der Wundheilung nützlich sind oder dazu verwendet werden.

3. Pharmazeutisches Produkt nach Anspruch 1 oder 2, **dadurch gekennzeichnet, dass** die Wirksubstanz(en) aus der Gruppe ausgelesen ist(sind), die aus Anästhetika vom Typ Aminoamid besteht, und insbesondere aus der Gruppe, die aus Lidocain, einem Lidocainsalz, einem Gemisch aus Lidocain oder einem Lidocainsalz und Prilocain oder einem Prilocainsalz, Articain und einem Articainsalz besteht.

4. Pharmazeutisches Produkt nach einem der Ansprüche 1 bis 3, **dadurch gekennzeichnet, dass** die Wirksubstanz(en) in einer Menge zwischen 45 Massen-% und 65 Massen-% in Bezug auf die gesamte Trockenmasse des Produkts vorliegt.

5. Pharmazeutisches Produkt nach einem der Ansprüche 1 bis 4, **dadurch gekennzeichnet, dass** es höchstens 15 Massen-% in Bezug auf seine gesamte Trockenmasse eines oder mehrerer Additive umfasst, die aus organischen Salzen, anorganischen Salzen, Säureregulatoren, Weichmachern, Feuchtigkeitsspendern und Farbkorrektoren ausgewählt sind.

6. Pharmazeutisches Produkt nach einem der Ansprüche 1 bis 5, **dadurch gekennzeichnet, dass** die Oberfläche des Produkts, die auf die Haut, ein Gewebe oder eine Schleimhaut aufgetragen oder angewendet werden soll, Mikronadeln aufweist, deren durchschnittliche Höhe zwischen 150 und 3000 µm liegt.

7. Pharmazeutisches Produkt nach einem der Ansprüche 1 bis 6, **dadurch gekennzeichnet, dass** es ein oder mehrere zusätzliche(s) Element(e) aufweist, das (die) aus der Gruppe ausgewählt sind, die aus einer Kunststofffolie, einem festen Kunststoffteil, einer gewobenen Textilie oder einer nicht-gewobenen Textilie besteht.

8. Verpackung, in der, unter Vakuum oder unter einem Druck, der unter dem Atmosphärendruck liegt, ein pharmazeutisches Produkt, nach einem der Ansprüche 1 bis 7, untergebracht ist.

9. Verfahren zur Herstellung eines pharmazeutisches Produkts nach einem der Ansprüche 1 bis 7, wobei das Verfahren die Schritte umfasst, die beruhen auf der:
a) Herstellung einer wässrigen Lösung, die zwischen 40 Massen-% und 65 Massen-% mindestens einer Wirksubstanz enthält, zwischen 25 Massen-% und 53 Massen-% Poly(vinyl)pyrrolidon (PVP) und zwischen 5 Massen-% und 30 Massen-% Carboxymethylcellulose (CMC) oder eines ihrer Salze, wobei die Massen-% in Bezug auf die gesamte Trockenmasse der Lösung ausgedrückt werden;
b) Anwendung der in Schritt a) hergestellten Lösung auf einem Träger; und
c) Trocknung der Lösung, wodurch ein pharmazeutisches Produkt erhalten wird.

10. Herstellungsverfahren nach Anspruch 9, **dadurch gekennzeichnet, dass** der Träger, der in Schritt b) eingesetzt wird, eine Form ist und besonders eine Silikonform.

11. Herstellungsverfahren nach Anspruch 10, **dadurch gekennzeichnet, dass** der Schritt b) darauf beruht, die Form mit der wässrigen Lösung zu füllen, die in Schritt a) hergestellt wurde, und ein Vakuum zwischen 50 und 500 mbar von unterhalb der Form anzuwenden.

12. Pharmazeutisches Produkt nach einem der Ansprüche 1 bis 7 zur Verwendung als Human- und/oder Tierarzneimittel.

13. Pharmazeutisches Produkt nach einem der Ansprüche 1 bis 7 zur Verwendung als Anästhetikum zur Anwendung auf der Haut, einem Gewebe oder einer Schleimhaut oder zur Unterstützung oder Verbesserung der Wundheilung, besonders zur Begrenzung der Zelladhäsion und der Bildung von fokalen Punkten im Bereich von Narben.

## Claims

1. A pharmaceutical product in the form of a film, disk, pellet or patch for application to the skin, tissue or a mucous membrane comprising:
- between 40% and 65% by mass of at least one active substance;
- between 25% and 53% by mass of poly(vinyl) pyrrolidone (PVP); and
- between 5% and 30% by mass of carboxymethylcellulose (CMC) or one of its salts;
the % by mass being expressed in relation to the total dry mass of said product.

2. The pharmaceutical product according to claim 1, **characterized in that** said active substance(s) is/are selected from the group consisting of active ingredients which are useful or used for anesthesia and/or to promote or improve healing.

3. The pharmaceutical product according to claim 1 or 2, **characterized in that** said active substance(s) is/are selected from the group consisting of amino-amide type anesthetics and, in particular, from the group consisting of lidocaine, a lidocaine salt, a mixture of lidocaine or a lidocaine salt and prilocaine or a prilocaine salt, articaine and an articaine salt.

4. The pharmaceutical product according to any one of claims 1 to 3, **characterized in that** said active substance(s) are present in an amount comprised between 45% and 65% by mass relative to the total dry mass of said product.

5. The pharmaceutical product according to any one of claims 1 to 4, **characterized in that** it comprises at most 15% by mass relative to its total dry mass of one or more additive(s) selected from organic salts, inorganic salts, acidity correctors, plasticizers, humectants and color correctors.

6. The pharmaceutical product according to any one of claims 1 to 5, **characterized in that** the surface of said product to be affixed or applied to the skin, tissue or a mucous membrane has micro-needles whose average height is comprised between 150 and 3000 µm.

7. The pharmaceutical product according to any one of claims 1 to 6, **characterized in that** it has one or more additional element(s) selected from the group consisting of a plastic film, a solid plastic part, a woven textile or a nonwoven textile.

8. A packaging wherein a pharmaceutical product as defined in any one of claims 1 to 7 is stored, under vacuum or under pressure below atmospheric pressure.

9. A method for preparing a pharmaceutical product as defined in any one of claims 1 to 7, said method comprising the steps of:
(a) preparing an aqueous solution containing between 40% and 65% by mass of at least one active substance, between 25% and 53% by mass of poly(vinyl) pyrrolidone (PVP) and between 5% and 30% by mass of carboxymethylcellulose (CMC) or one of its salts, the % by mass being expressed relative to the total dry mass of said solution;
b) applying said solution prepared in step a) to a support and
c) drying said solution whereby a pharmaceutical product is obtained.

10. The preparation method according to claim 9, **characterized in that** said support used during step b) is a mold and in particular a silicone mold.

11. The preparation method according to claim 10, **characterized in that** said step b) consists in filling said mold with the aqueous solution prepared during step a) and in applying a vacuum comprised between 50 and 500 mbar from below the mold.

12. The pharmaceutical product as defined in any one of claims 1 to 7, for use as a medicament for human and/or veterinary use.

13. The pharmaceutical product as defined in any one of claims 1 to 7, for use as an anesthetic agent to be applied to the skin, tissue or a mucous membrane or to promote or improve healing, in particular to limit tissue adhesion and the formation of focal points at the scars.
